# EUROPÄISCHE PATENTANMELDUNG

(11) **EP 2 103 309 A2**
(43) Veröffentlichungstag der Anmeldung: **23.09.2009**
(21) Anmeldenummer: 09151539.5
(22) Anmeldetag: 28.01.2009
(51) Int. Cl.: A61K 36/00, A61K 35/00

(54) **Verfahren zur Extraktion und zum Nachweis von fettlöslichen Inhaltsstoffen aus biologischen Materialien**

(30) Priorität: 19.03.2008 EP 08005142
(71) Anmelder: SCHWEIGERT, Florian, 12205 Berlin (DE)
(72) Erfinder: SCHWEIGERT, Florian, 12205 Berlin (DE)
(74) Vertreter: Schwander, Kuno

(57) **Zusammenfassung**

Die Erfindung betrifft ein Verfahren zur Analyse von fettlöslichen Inhaltsstoffen, insbesondere von fettlöslichen Farbstoffen aus biologischer Materialien insbesondere Nahrungs- und Futttermittel mit einer erleichterten Herauslösung der fettlöslichen Inhaltsstoffe aus den biologischen Materialien unter Verwendung geeigneter Verdünnungslösungen und der Extrahierbarkeit unter Verwendungen diesbezüglicher organischer Lösungsmittel oder organischer Lösungsmittelgemische sowie eins Anreicherungs- und Trennverfahrens ,mit anschließender digitaler Auswertung und Dokumentation. Es wird vorgeschlagen, die biologischen Materialien zunächst mit einem Verdünnungsmittel zu behandeln das die fettlöslichen Inhaltsstoffe leichter aus der komplexen biologischen Matrix extrahierbar macht und anschließend mit mindestens einem organischen Lösungsmittel zu behandeln, welches die Inhaltsstoffe extrahiert: Die in den organischen Überstand extrahierten Substanzen werden abschließend chromatographisch angereichert und getrennt und dann visuell beurteilt und/oder vermessen.

## Beschreibung

Die vorliegende Erfindung betrifft ein Verfahren zur Analyse von fettlöslichen Inhaltsstoffen, insbesondere Farbstoffen, aus biologische Materialien, insbesondere Nahrungsmitteln, mit einer Anreicherung der Inhaltsstoffe und nachfolgender Analytik. Das Verfahren besteht insbesondere aus einer Kombination von Extraktions- und Separationsschritten und einem nachfolgenden Analyse-Schritt.
Die Erfindung betrifft ferner Analyse-Kits und Analysegeräte zur Durchführung des Verfahrens.

Das erfindungsgemässe Verfahren setzt sich aus mehreren Schritten zusammen. Dei zwei wesentlichen und die Erfindung kennzeichnende Schritte sind:
- Aufnahme einer Probe des biologischen Materials in ein Lösungsmittel, das die Extrahierbarkeit von fettlöslichen Inhaltstoffen erleichtert (Probenvorbereitungsschritt).
- Extrahierung der fettlöslichen Inhaltstoffen in ein Extraktionsgemisch mittels eines organischen Lösungsmittels oder Lösungsmittelgemisches bei gleichzeitiger Abtrennung der nicht fettlöslichen Komponenten sowie der Optimierung des Extraktionsverhältnisses von natürlich vorkommenden Inhaltstoffen und den zu untersuchenden fettlöslichen Inhaltstoffen (Extraktionsschritt).

Abhängig von der zu analysierenden Substanz kann der extrahierte Inhaltsstoff noch vor der Analytik weiter augetrennt werden.

Der Erfindung steht die Auflösung der komplexen Verbindung zwischen den fettlöslichen Inhaltsstoffen und der biologischen Matrix, die Bereitstellung von Analyse-Kits und die Anwendung des Verfahrens zur Bestimmung der nachfolgend auch als Inhaltsstoffe bezeichneten Substanzen in Lebensmitteln und Futtermittel im Vordergrund.

Für das erfindungsgemässe Nachweisverfahren in Frage kommende fettlösliche Inhaltsstoffe sind Lipide und Lipoide. Diese Gruppe von Substanzen ist gekennzeichnet durch ihre fehlende Löslichkeit in Wasser oder wässrigen Lösungen und ihre gute Löslichkeit in organischen Lösungsmitteln. Man spricht von Lipophilität. Zu diesen Substanzen gehören neben den wesentlichen Fetten wie Triglyzeriden, Phospholipiden und Cholesterin eine Vielzahl von Substanzen die zum Teil in sehr geringen Mengen anzutreffen sind und sich hinsichtlich ihrer chemischen Struktur sehr unterscheiden. Dazu gehören fettlösliche Vitamin wie die Retinoide, Vitamin E, Vitamin D und Vitamin K. Weiter Substanzen sind Steroide wie beispielsweise Hormone, Pherhormone und Mykotoxine. Weiter Substanzen die für das Nachweisverfahren in Frage kommen sind lipohile Farbstoffe natürlichen oder synthetischen Ursprungs. Dies betrifft beispielsweise die Gruppe der Carotinoide (Beta-Carotin, Alpha-Carotin, Canthaxanthin, Astaxanthin, Lutein, Lycopen) als Bestanteil zahlreicher pflanzlicher oder tierischer Produkte oder synthetische (künstliche) Farbstoffe der Azo-Gruppe (Sudan G, Sudan Braun, Sudan R, Citrus Red Nr 2, Sudan Gelb GRN, Sudan II, Oil Red O, Chinolingelb SS, Alizarin Violett 3 B, Solvent Blau 35, Quinizarine Green SS).

Natürliche und synthetische Farbstoffe werden zur Einfärbung von Produkten unterschiedlicher Anwendung eingesetzt. Sie dienen dazu bestimmte optische Qualitätsmerkmale zu vermitteln. Biologische Materialien die eingefärbt werden können sind Eidotter und Eiprodukte, Gewürze, Gewürzmischungen und Gewürzzubereitungen in fester pastöser oder flüssiger Form, Fleisch und Fleischprodukte, Fisch- und Fischprodukte, Obst-, Frucht- und Gemüsesäfte bzw. Zubereitungen sowie Butter oder andere Milchprodukte. Die Einfärbung der zum menschlichen Verzehr eingesetzten biologischen Materialen kann entweder über die Fütterung oder im Verarbeitungsprozess eingebracht werden.

Eingefärbten Produkte betreffen nicht nur Nahrungs- und Futtermittel sondern auch technische Produkte und beispielsweise Kosmetikprodukte.

Lipide und Lipoide sind in biologischen Membranen assoziert oder an spezifische oder unspezifische Proteine gebunden. Ein Beispiel ist die Anreicherung von Lipiden oder Lipoiden wie Vitamin E oder Phospholipiden in Zellmembranen wird für Zellen von Tieren und dem Menschen beschrieben. Diese Bindung und die Lipophilität setzen besondere Ansprüche an die Extraktion. Auch die Einlagerung in Zellmembranen oder andere Zellstrukturen in tierischen oder pflanzlichen biologischen Matrixes stellt ein Extraktionsproblem dar.

Moderne Verfahren zur Analyse von biologischen Materialien weisen oftmals Schritte zur Abtrennung, Extraktion, Isolation und/oder Anreicherung von Bestandteilen sowie Inhaltsstoffen der biologischen Materialien auf. Solche Verfahrensschritte sind sowohl in der qualitativen als auch quantitativen Analytik zur Abtrennung von störenden oder ergebnisverfälschenden Substanzen unumgänglich.

Die bekannten Nachweisverfahren sind darüber hinaus zeitaufwendig und geräteintensiv, dies vor allem wenn sehr geringe Mengen der Inhaltsstoffe nachgewiesen werden sollen. Andererseits führen deutliche Vereinfachungen der bekannten Verfahren in der Regel dazu, dass nur sehr große Konzentrationen nachgewiesen werden können.

Erhöhte Anforderungen an die Unbedenklichkeit von Nahrungs- und Futtermitteln bzw. der Nachweis von toxischen Substanzen oder verfälschenden Substanzen machen vor allem im internationalen Warenverkehr rasche, sensitive und zuverlässige Analysen, vorzugsweise möglichst nahe am Produkt, notwendig. Deshalb sind Verfahren die aus möglichst wenigen Arbeitsschritten bestehen und/oder einen geringen technischen Aufwand erfordern notwendig. Wünschenswert ist dabei der Einsatz von Verfahren die auf der Basis von Einweg-Analyse-Kits angewendet werden können.

In diesem Zusammenhang steht beispielsweise der Nachweis von Farbverfälschung von Nahrungs- und Futtermitteln mit Sudan Rot oder der Nachweis von ebenfalls toxischen Mykotoxinen.

Ziel der Erfindung ist daher ein Verfahren, eine Extraktion von endogenen oder exogenen fettlöslichen Stoffen bereitzustellen, welches selektiv genug ist, die Stoffe mit vergleichsweise geringem Aufwand aus biologischem Material, beispielsweise einer komplexen festen Matrix zu lösen, und im Bedarfsfall mittels eines Einweg-Analyse-Kits einfach und schnell angewandt werden kann. Gleichzeitig soll mit einem Verdünnungspuffer möglichst viele unterschiedliche Extraktionsprobleme gelöst werden können, so dass das Verfahren beispielsweise zum Nachweis der Konzentration von Lipiden und lipophilen Substanzen natürlichen oder synthetischen Ursprungs Anwendung findet.

Dieses Ziel wird durch das Verfahren gemäss Anspruch 1, die Verwendung gemäss Anspruch 9, Analyse-Kits gemäss Anspruch 13 und optische Analysegeräte gemäss Anspruch 15 gelöst.

Bevorzugte Ausführungsformen sind in den abhängigen Ansprüchen dargelegt. Der Wortlaut sämtlicher Ansprüche wird hiermit durch Bezugnahme zum Inhalt dieser Beschreibung gemacht.

Das Verfahren gemäß der vorliegenden Erfindung ist zur Analyse von fettlöslichen Inhaltsstoffen, d.h. Lipiden und/oder Lipoiden vorzugsweise natürliche und synthetische lipophile Inhaltsstoffe wie Carotinoide, Vitamine, Hormone, Farbstoffe oder Mykotoxine aus festen biologischen Materialien vorgesehen. Insbesondere dient das Verfahren einer Extraktion von lipophilen Inhaltsstoffen. Die biologischen Materialien werden erfindungsgemäss zunächst so vorbehandelt, dass es zu einer leichten Abtrennung der lipophilen Substanzen aus ihrer Bindungsform im wässrigen Milieu kommt. Sie können dann mit organischen Lösungsmitteln behandelt werden. Dabei werden die Inhaltsstoffe in das Extraktionsmittel überführt und extrahiert.

Vorbehandlung, Extraktion und allenfalls anschliessende Separation sind erfindungsgemäss so kombiniert, dass die Substanzen in sehr geringer Menge nachgewiesen werden können. Grenzwerte die bis dato nur über die HPLC erreicht werden, können so durch die Kombination einfachster Trenn- und Extraktionsmethoden erreicht werden.

Als Vorbehandlung dient ein spezifischer Verdünnungsschritt (mittels einer Salz- oder Pufferlösung), der gegebenenfalls, d.h. vor allem bei festen biologischen Materialien, noch zusätzlich mit einem Aufschluss-Schritt kombiniert wird. Als Aufschlüsse kommen Behandlungen mit Homogenisatoren in Frage. Als Homogenisatoren sind beispielsweise Cutter, Ultraturrax-Geräte, Mühlen usw. zu nennen. Die biologischen Materialien werden hierdurch einer Zerkleinerung unterworfen, um ihre Oberfläche zu vergrößern. Durch die Zerkleinerung wird die Extraktion in ihrer Effizienz gesteigert. Daneben kommen auch Behandlungen mit Kälte, insbesondere mit flüssigem Stickstoff, in Frage. Die Aufschlüsse dienen dazu, Inhaltsstoffe aus den biologischen Materialien freizusetzen, und somit einer Extraktion zugängig zu machen. Die biologischen Materialien können als Vorbehandlung auch einer Abtrennung von Verunreinigungen und/oder störenden Substanzen, insbesondere Waschvorgängen, beispielsweise mit Pufferlösungen, unterworfen werden.

Überraschenderweise wurde gefunden, dass durch den Einsatz verschiedener Lösungen wie beispielsweise Puffer- oder Salzlösungen die Extraktionseffizienz gegenüber reinem Wasser von Lipiden und lipophilen Substanzen in ein organisches Lösungsmittel deutlich verbessert wird. Dies beruht unter anderem auf einer effizienteren Auflösung der komplexen Matrix biologischer Materialien.

Die vorliegende Erfindung löst daher das ihr zugrundeliegende technische Problem durch die Verwendung verschiedener Puffer- oder Salzlösungen zur Auflösung komplexer Matrixstrukturen im Rahmen der Probenvorbereitung für eine spätere effizientere Extraktion der fettlöslichen Inhaltsstoffe. Das beschriebene Verfahren verbessert die Effizienz der Extraktion vor allem fettlöslichen Inhaltsstoffen aus verschiedenen biologischen Materialien vorzugsweise biologischer Materialien die in der Ernährung von Mensch und Tier verwendet werden.

Im Rahmen der hier vorliegenden Erfindung sollen unter "biologischen Materialien" von Pflanzen, Tieren oder Mikroorganismen gewonnene Materialien, insbesondere körpereigene Materialien, vorzugsweise Proben von körpereigenen Materialien, verstanden werden.

Bei dem körpereigenen Material handelt es sich bevorzugt um Material, das aus dem lebenden Organismus entnommen wurde, aber auch die post mortem Entnahme ist erfinderisch vorgesehen. In einer bevorzugten Ausführungsform handelt es sich bei den körpereigenen Materialien um Körperflüssigkeiten, Gewebe und/oder Organe. Dabei können die Körperflüssigkeiten Blut, Plasma, Serum, Harn, Amnionflüssigkeit, Milch, Uterusflüssigkeit, Follikelflüssigkeit, Liquor, Synovia, Tränenflüssigkeit, Pankreassekret, Magensaft und/oder Speichel sowie alle anderen Körperflüssigkeiten physiologischer oder pathologischer Natur sein. Die Körperflüssigkeiten können durch Punktion bzw, mittels etablierte Sammelverfahren am lebenden Organismus oder post-mortem gewonnen werden. Das Blut kann dabei z. B. durch Punktion von Blutgefäßen (Venenpunktion) gewonnen werden. Die Blutentnahme bzw. die Plasma- und Serumgewinnung sowie die Gewinnung anderer Körperflüssigkeiten erfolgt nach etablierten Verfahren. Sie beinhalten die Punktion der Gefäße, Trennung der Blutbestandteile, vorzugsweise durch Zentrifugation, und die Lagerung, vorzugsweise bei - 80°C. Im Fall einer unmittelbaren Analyse ist die Probe vorzugsweise bis zur Analyse bei 4°C zu lagern. Gewebe und Organe werden durch etablierte Biopsieverfahren oder post mortem entnommen und mit entsprechend etablierten Verfahren gelagert. Vor der Analyse müssen die Organe und Gewebe zunächst beispielsweise nach etablierten Verfahren in entsprechenden Puffer homogenisiert werden. Als Puffer bietet sich beispielsweise Tris-HCl (pH 7,8) an. Methoden zur Herstellung von Gewebehomogenaten sind dem Fachmann bekannt.

Unter "Mikroorganismen" sollen im Rahmen der hier vorliegenden Erfindung neben beispielsweise Eukaryoten, wie Algen, Prokaryoten und/oder Pilzen, wie Hefen, auch Viren umfasst sein. Insbesondere sollen "biologische Materialien" aus Kultur gewonnene Organismen sowie Kulturüberstände umfassen.

Erfindungsgemäß können alle biologischen Materialien verwendet werden, welche der Extraktion zugeführt werden können.

In einer besonders bevorzugten Ausführungsform des Verfahrens werden als biologische Materialien Nahrungs- und Futtermittel tierischen und/oder pflanzlichen Ursprungs, insbesondere Homogenisate von Lebensmitteln, eingesetzt.

Bevorzugterweise kommen für das erfindungsgemäße Verfahren Lebensmittel in Betracht, welche für die Ernährung von Menschen essentielle Inhaltsstoffe, vorzugsweise lipophile Inhaltsstoffe, aufweisen.

Unter Produkten die von Tieren oder Pflanzen stammen sollen im Rahmen der hier vorliegenden Erfindung beispielsweise Eidotter und Eiprodukte, Gewürze, Gewürzmischungen und Gewürzzubereitungen in fester pastöser oder flüssiger Form, Fleisch und Fleischprodukte, Fisch- und Fischprodukte, Obst-, Frucht- und Gemüsesäfte bzw. Zubereitungen sowie Butter oder andere Milchprodukte

Bevorzugerweise werden auch solche biologischen Materialien verwendet, welche im medizinischen Bereich für eine Diagnostik und/oder Therapieverfolgung eingesetzt werden.

In einer weiteren bevorzugten Ausführungsform des Verfahrens werden als Körperflüssigkeiten Blut, Plasma, Serum, Harn, Amnionflüssigkeit, Uterusflüssigkeit, Folikelflüssigkeit, Synovia, Sperma, Lungenflüssigkeit und/oder Sekrete verwendet.

Bevorzugterweise handelt es sich bei den Sekreten um Milch, Schweiß, Tränenflüssigkeit, Speichel und/oder Magen-Darm-Trakt-Sekrete, insbesondere um Gallenflüssigkeiten und/oder Pankreassekret. Erfindungsgemäß kann als Körperflüssigkeit Blut, vorzugsweise Vollblut, verwendet werden. Bevorzugterweise wird als biologisches Material im erfindungsgemäßen Verfahren Blut verwendet. Als Vorbehandlung wird das Blut bevorzugterweise mit gerinnungshemmenden Stoffen behandelt, insbesondere mit polyanionischen Polysacchariden, vorzugsweise mit Heparin und/oder Heparinoiden. Weiterhin können als Antikoagulanzien Anti-Thrombin III, insbesondere in Form von Heparin-Anti-Thrombin-Komplexen, eingesetzt werden. Weiterhin können auch körperfremde Antikoagulanzien eingesetzt werden. Als körperfremde Antikoagulanzien kommen insbesondere Vitamin-K-Antagonisten oder Kalzium-Komplexbildner in Frage. Als Vitamin-K-Antagonisten sind insbesondere Cumarine zu nennen, als Kalziumkomplexbildner sind insbesondere Citrat, Oxalat, vorzugsweise Ethylendiamintetraacetat (EDTA), zu nennen.

Vorteilhafterweise wird durch das erfindungsgemäße Verfahren eine Hämolyse des Blutes, insbesondere der Blutzellen, vermieden. Durch die erfindungsgemäße Verfestigung des Blutes werden die Blutzellen in einer gelartigen Umgebung eingebettet und somit vor einer Hämolyse geschützt.

In einer weiteren Ausführungsform umfassen die biologischen Materialien pflanzliche, tierische, menschliche und/oder mikrobielle Materialien, welche insbesondere aus Zellkulturen stammen.

In einer weiteren bevorzugten Ausführungsform des Verfahrens werden die biologischen Materialien vor ihrem Einsatz mechanischen Aufschlüssen und Reinigungsvorgängen, unterworfen.

Zur Bereitstellung der biologischen Materialien werden im erfindungsgemäßen Verfahren Proben der biologischen Materialien eingesetzt, bevorzugt von Menschen oder tierischen Organismen entnommene Proben. Zweckmäßigerweise können auch abgegebene bzw. sezernierte biologische Materialien verwendet werden. Weiterhin ist es auch möglich, die biologischen Materialien vor ihrer Verwendung im erfindungsgemäßen Verfahren zu kultivieren, insbesondere außerhalb vom menschlichen oder tierischen Körper.

Vorteilhaft ist ferner, daß die Extraktion manuell durch Schütteln, insbesondere vorsichtiges, eine Hämolyse vermeidendes Schütteln, durchgeführt wird. Für die Extraktion des Blutes mit den Lösungsmitteln können auch Hilfsmittel, insbesondere Rütteltische, Schwenkwippen, Überkopfschüttler, Magnetrührer und andere Rührtechniken eingesetzt werden. Ein manuelles Mischen hat den Vorteil, von einer Stromquelle oder von elektrischen Geräten unabhängig die Extraktion vornehmen zu können.

Ziel der Zugabe einer Verdünnungslösung bei der Probenvorbereitung ist nicht nur die Verdünnung der Probe sondern vor allem die Vorbereitung zur Extraktion durch eine Auf- oder Anlösung der komplexen Matrix. Optimalerweise verändert die Verdünnungslösung die Probe derart, dass Inhaltsstoffe leichter, gezielt und vollständiger extrahiert werden können. Dabei spielen beispielsweise Protein-Wechselwirkungen mit den Inhaltsstoffen der Verdünnungslösung eine Rolle. Diese Wirkung beruht entweder auf einer generellen Erhöhung der Ionenkonzentration gegenüber reinem Wasser oder des Eintrags spezifischer Inhaltsstoffe die mit Komponenten der Matrix reagieren. Dies betrifft die Lösung von Disulfied Bindungen, die Entfaltung von Proteinen durch stark hygroskopische Moleküle oder die Wechselwirkung mit Phosphatgruppen. Auch die Auflösung von komplexen chemischen Strukturen durch Enzyme ist im sinne des erfinderischen Schrittes möglich.

Überraschenderweise wurde gefunden, dass eine Vorbehandlung mit bestimmten Salz- oder Pufferlösungen insbesondere mit einer Harnstofflösungen unterschiedlicher Konzentration zu einer deutlichen Verbesserung der komplexen Strukturen biologischer Matrix und damit einer effizienteren Extraktion führt.

Salz- und Pufferlösungen er Lösungen aus organischen Verbindungen können im Rahmen der hier vorliegenden Erfindung entweder aus nur einer Komponente bestehen oder aus einer Mischung von mindestens zwei unterschiedlichen Komponenten.

Auch ein Zusatz von wasserlöslichen organischen Komponenten, Detergentien, Tensiden insbesondere nicht-ionische Tenside oder Emulgatoren können die Extraktionseffizienz fördern. Als Zusätze kommen beispielsweise DMSO oder DTT in Frage.

Auch ein Zusatz von Enzymen wie beispielsweise Proteasen oder Lipasen ist vorgesehen um entweder die Komplexizität der Matrix zu verringern - wie beispielsweise im Falle der Lipasen - oder um störende Begleitkomponenten zu entfernen - wie beispielsweise durch die Lipasen.

Bei der Verwendung von Harnstoff in einem Konzentrationsbereich von 0.1 bis 8 M vorzugsweise 1 bis 8 M und im speziellen 2 bis 4 M reicht bei biologischen Materialien wie Eiern, Fischmuskulatur oder Leber entweder nur ein einfaches Schütteln bzw. der Einsatz eines Handmixers von der Drehzahl und Kraftentwicklung eines Milchaufschäumers aus. Dadurch können komplexe Extraktionsverfahren deutlich verbessert und erleichtert werden.

Auf Grund unterschiedlicher physiko-chemischer Eigenschaften fettlöslicher Komponenten und die großen Unterschiede in die Matrix der biologischen Probe ist die jeweilige Zusammensetzung eines Lösungsmittels für die Auflösung der Probenmatrix für nachfolgende Extraktion von großer Bedeutung.
Weitere Lösungsmittel zur Porbenvorbereitung bzw. Probenverdünnung lassen sich abhängig vom eingesetzten biologischen Material wie folgt beschreiben bzw.. definieren:

Unter Puffer sollen im Rahmen der hier vorliegenden Erfindung Pufferlösung beziehungsweise ein Puffersystem, dh. Ein ein Stoffgemisch, dessen pH-Wert (Konzentration der Wasserstoffionen) sich bei Zugabe einer Säure oder Base wesentlich weniger stark ändert, als dies in einem ungepufferten System der Fall wäre, umfasst sein.Solche Pufferlösungen enthalten eine Mischung aus einer schwachen Säure und ihrer konjugierten Base (oder des jeweiligen Salzes).

Auch Ampholyte und bifunktionale Moleküle können als Puffer dienen. Der den pH-Wert bestimmende Faktor ist das Verhältnis bzw. das Protolyse-Gleichgewicht des Pufferpaares.

Beispiele für Pufferlösungen sind: Essigsäure/Acetat-Puffer, Phosphatpuffer KH₂PO₄ + Na₂HPO_{4;} Veronal-Acetat-Puffer nach Michaelis; Ammoniakpuffer NH₃ + H₂O + NH₄Cl; HEPES (4-(2-Hydroxyethyl)-1-piperazinethanesulfonsäure) PBS-Puffer; MES (2-(N-Morpholino)ethansulfonsäure).

Salzlösungen sind Lösungen aus Salze, die aus positiv geladenen Ionen, den sogenannten Kationen und negativ geladenen Ionen, den sogenannten Anionen aufgebaut sind. Salze organischer oder anorganischer Natur sein. Im engsten Sinn versteht man unter Salz das Natriumchlorid (NaCl, Speisesalz). Im weiten Sinn bezeichnet man alle Verbindungen als Salze, die wie NaCl aus Anionen und Kationen aufgebaut sind.

Als komplexe Salze bezeichnet man Salze, bei denen unter Mitwirkung von Molekülen eigenständige (stabile) Ionen vorliegen.

Neben Salzen mit nur einer Art von Kationen, sind auch Salze mit zwei verschiedenen Kationen bekannt. Man nennt diese Salze Doppelsalze, wie die Alaune mit der allgemeinen Zusammensetzung M^{I}M^{III}(SO₄)₂. Ein Beispiel ist das Aluminiumkaliumsulfat-Dodecahydrat (KAl(SO₄)₂ · 12 H₂O).

Neben den beschriebenen anorganischen Salzen gibt es auch Salze organischer Verbindungen. Die Anionen dieser Salze stammen von den organischen Säuren ab. Wichtig sind hier die Salze der Carbonsäuren, wie beispielsweise die Essigsäure, von der viele Salze, die so genannten Acetate (CH₃COO⁻) bekannt sind. Beispiele sind die Salze Natriumcitrat und Calciumcitrat.
Unter "organischen Verbindungen die eine Auflösung von komplexen Matrixes" im Sinne der vorliegenden Erfindung erfüllen, fällt beispielsweise der Harnstoff.
Harnstoff stammt aus dem Protein- und Aminosäurestoffwechsel von Mensch- und Tier. Harnstoff wird wegen seiner hohen Wasserbindungsfähigkeit als Keratolytikum eingesetzt, das komplexe Matrixes auflöst. Er wird Lebensmitteln als Stabilisator zugesetzt. In der EU ist er als Lebensmittelzusatzstoff mit der Bezeichnung E 927b ausschließlich für Kaugummi ohne Zuckerzusatz zugelassen.

Als organische Lösungsmittel zur Extraktion und allenfalls anschliessender Analytik werden polar protische Lösungsmittel verwendet, insbesondere Alkohole.

In einer weiteren bevorzugten Ausführungsform des Verfahrens sind die polar protischen Lösungsmittel ausgewählt aus der Gruppe, umfassend Methanol, Ethanol, 1-Propanol, 2-Propanol (Isopropanol), Butanol, Pentanol, Hexanol sowie Gemische davon. Bevorzugt werden dabei Gemische von Ethanol und 2-Propanol (Isopropanol) verwendet.

In einer weiteren bevorzugten Ausführungsform des Verfahrens wird als organisches Lösungsmittel mindestens ein polar aprotisches Lösungsmittel verwendet, insbesondere werden Ester, vorzugsweise Essigsäureethylester, verwendet.

Vorteilhafterweise lassen sich im erfindungsgemäßen Verfahren neben den bevorzugten polar protischen Lösungsmitteln auch polar aprotische Lösungsmittel einsetzen.

In einer weiteren Ausführungsform des Verfahrens werden als polar aprotische Lösungsmittel Nitrile, vorzugsweise Acetonitril, verwendet.

In einer weiteren Ausführungsform des Verfahrens werden als polar aprotische Lösungsmittel Ketone, vorzugsweise Aceton, verwendet.

In einer weiteren Ausführungsform des Verfahrens werden als polar aprotische Lösungsmittel Dimethylsulfoxid und/oder N,N-Dimethylformamid verwendet.

In einer weiteren Ausführungsform des Verfahrens werden als polar aprotische Lösungsmittel Ether, insbesondere Diethylether, verwendet.

Bevorzugterweise werden die polaren Lösungsmittel abhängig von den biologischen Materialien in Form von Gemischen eingesetzt.

In einer weiteren bevorzugten Ausführungsform des Extraktionsschrittes werden als Lösungsmittel mindestens ein unpolares Lösungsmittel, insbesondere Alkane, vorzugsweise C5- bis C12-Alkane, verwendet.

In einer weiteren bevorzugten Ausführungsform des Extraktionsschrittes werden als unpolare Lösungsmittel Hexan, Heptan und/oder Octan, insbesondere Isooctan, verwendet.

In einer weiteren Ausführungsform des Extraktionsschrittes werden als unpolare Lösungsmittel Aromaten, insbesondere Toluol und/oder Benzol, verwendet.

Die genannten unpolaren Lösungsmittel dienen im erfindungsgemäßen Verfahren als Extraktionsmittel für die Inhaltsstoffe, insbesondere für die lipophilen Inhaltsstoffe.

Erfindungsgemäß kann es zweckmäßig sein Derivate von organischen Lösungsmittelmolekülen zu verwenden. Die Lösungsmittel können wasserfrei, wasserhaltig, verzweigt, unverzweigt, cyclisch, acyclisch, halogeniert oder unhalogeniert sein.

Eine Einteilung in polare oder unpolare Lösungsmittel kann in der Technik unter verschiedenen Gesichtspunkten geschehen. Beispielsweise können aus der Chemie bekannte Definitionen von Polarität oder Lösungsmittelverhalten angewendet werden.

Daneben findet in der Praxis ein Polaritätsindex nach Snyder oder Keller Verwendung (Snyder, Principles of absorption chromatography, Decker, New York, 1968; Keller, Analytical chemistry, Weinheim, 1998, Seite 195), um Lösungsmittel oder Lösungsmittelgemische zu klassifizieren. Danach wird unter polaren Lösungsmitteln oder Lösungsmittelgemischen ein Lösungsmittel oder Lösungsmittelgemisch mit einem Polaritätsindex von 4 bis 8, insbesondere von 5 bis 7, vorzugsweise von 5,5 bis 6,5, nach Snyder verstanden. Polare Lösungsmittel sind beispielsweise Wasser, insbesondere wäßrige Lösungen. Polar aprotische Lösungsmittel sind beispielsweise Aceton, Acetonitril, Essigsäureethylester, Dimethylsulfoxid oder N,N-Dimethylformamid. Polar protische Lösungsmittel sind beispielsweise Alkohole, welche einen Alkylrest mit 1 bis 6 Kohlenstoffatomen aufweisen, beispielsweise Methanol, Ethanol, 1-Propanol, 2-Propanol (Isopropanol), Buthanol, Pentanol oder Hexanol.

Unter einem unpolaren Lösungsmittel oder unpolaren Lösungsmittelgemisch wird ein Lösungsmittel oder Lösungsmittelgemisch verstanden, welches im Vergleich zu einem Referenzlösungsmittel oder Referenzlösungsmittelgemisch einen um 0,3 oder mehr kleineren Polaritätsindex aufweist. Bevorzugt ist ein um 0,5 kleinerer Polaritätsindex, insbesondere ein um 1 kleinerer Polaritätsindex, vorzugsweise ein um mehr als 2 kleinerer Polaritätsindex. Folglich weist der Polaritätsindex des unpolaren Lösungsmittels oder Lösungsmittelgemischs einen Wert von 5 bis 1, insbesondere von 4 bis 2, vorzugsweise von 3,5 bis 2,5, gemäß Snyder auf. Ein Lösungsmittelgemisch aus 60 % Methanol/40 % Dichlormethan hat beispielsweise einen Polaritätsindex von 3,1 nach Snyder. Als unpolare Lösungsmittel kommen demnach beispielsweise halogenhaltige Lösungsmittel wie Chloroform, Dichlormethan oder Tetrachlorkohlenstoff in Frage. Ferner sind aliphatische Lösungsmittel wie Pentan, Hexan, Heptan oder Cyclohexan zu nennen. Daneben sind als unpolare Lösungsmittel aromatische Lösungsmittel wie Toluol oder Benzol zu nennen. Weiterhin kommen Ether wie Diethylether, tert.-Butylmethylether oder Tetrahydrofuran in Frage.

In einer weiteren bevorzugten Ausführungsform des erfindungsgemässen Verfahrens werden die Lösungsmittel des Extraktionsschrittes in Form von Lösungsmittelgemischen eingesetzt, insbesondere in Form von Lösungsmittelgemischen, welche polare und unpolarische Lösungsmittel umfassen.

Bevorzugterweise werden die polaren und unpolaren Lösungsmittel in einem Verhältnis von 1:1, insbesondere in einem Verhältnis von 1:2, vorzugsweise in einem Verhältnis von 1:10 (polar:unpolar), eingesetzt.

Diese Maßnahme hat den Vorteil, daß je nach Beschaffenheit der biologischen Materialien Lösungsmittelgemische angepaßt hergestellt werden können. Auf diese Weise können eine Vielzahl von Trennproblemen bearbeitet werden.

Bei an die Extraktion anschließenden notwendigen Anreicherungs- und Trennschritten beispielsweise durch miniaturisierte Chromatographieverfahren kann es zweckmäßig sein, die Zusammensetzung der Extraktionslösungsmittel so zu wählen, dass es zu keiner Mischung bzw vollständigen Entmischung von beispielsweise Alkoholen und organischem Lösungsmittel kommt. Geeignet ist hier vorzugsweise DMSO (Dimethylsulfoxide) als aprotisches dipolares Lösungsmittel und n-Hexan oder iso-Octan. In diesem Fall kann das unpolarere Lösungsmittel auch dem Verarbeitungspuffer zugesetzt werden. Dadurch kann das extrahierende Lösungsmittel gleich als Laufmittel für die nachfolgende chromatographische Anreicherung und Trennung verwendet werden.

In einer weiteren bevorzugten Ausführungsform des Extraktionsschrittes werden ferner Tenside, insbesondere nichtionische Tenside, eingesetzt.

In einer weiteren bevorzugten Ausführungsform des Extraktionsschrittes werden als Tenside Copolymere, insbesondere Copolymere aus Polyethylenoxiden und Polypropylenoxiden, eingesetzt.

Bevorzugterweise werden solche Tenside verwendet, welche in dem Lösungsmittelgemisch löslich, frei von toxischen Eigenschaften sind und/oder die Analytik der im Überstand befindlichen Inhaltsstoffe unbeeinflußt lassen. Vorzugsweise bleiben Absorption und/oder Fluoreszenz der Inhaltsstoffe unverschlechtert meßbar.

Bevorzugterweise werden die Tenside in Konzentrationen eingesetzt, welche die Hämolyse von Blut ausschließen. Vorteilhafterweise werden als Tenside Copolymere, insbesondere Copolymere aus Polyethylenoxiden und Polypropylenoxiden, eingesetzt. Als Beispiel für Copolymer-Tenside kommen handelsübliche Pluronic-Tenside in Frage, vorzugsweise Pluronic 101.

In einer weiteren bevorzugten Ausführungsform werden die biologischen Materialien mit den organischen Lösungsmitteln in einem Verhältnis von 1:50, insbesondere in einem Verhältnis von 1:10, vorzugsweise in einem Verhältnis von 1:3, behandelt.

Je nach Beschaffenheit der biologischen Materialien und Extraktionsvermögen der organischen Lösungsmittel kann es zweckmäßig sein, ein größeres oder kleineres Verhältnis von biologischen Materialien zu organischen Lösungsmitteln zu wählen, insbesondere hängt dies von der nachfolgenden Analytik ab. Vorzugsweise wird das Verhältnis so gewählt, daß Nachweis- bzw. Bestimmungsgrenze in der Analytik der Inhaltsstoffe berücksichtigt werden.

In einer weiteren Ausführungsform wird das Verfahren bei einer Temperatur im Bereich von 5 °C bis 60 °C, insbesondere im Bereich von 10 °C bis 40 °C, durchgeführt.

In einer weiteren Ausführungsform wird das Verfahren bei einem Druck zwischen 0,5 bar bis 5 bar, insbesondere zwischen 0,8 bar bis 2 bar, durchgeführt.

Theoretisch läßt sich das Verfahren in Temperatur- und/oder Druckbereichen durchführen, bei welchen eine Gelbildung zu erwarten ist. Daneben sind Lösungsmitteleigenschaften, insbesondere Schmelzpunkt, Siedepunkt, Flammpunkt, zu berücksichtigen. Erfindungsgemäß wird das Verfahren bei Raumtemperatur und Normaldruck durchgeführt.

In einer weiteren bevorzugten Ausführungsform des Verfahrens werden die biologischen Materialien zur Extraktion der Inhaltsstoffe während eines Zeitraumes von 10 Sekunden bis 10 Minuten, insbesondere von 10 Sekunden bis 5 Minuten, vorzugsweise von 10 Sekunden bis 3 Minuten, behandelt.

Bevorzugterweise werden die Proben vor der Extraktion mit einem Verdünnungspuffer vorbehandelt. Das Verdünnungsverhältnis liegt zwischen 1:9 (Puffer:Probe) und 100:1, insbesondere 1:1 zu 50:1 vorzugsweise 10:1.

Bevorzugterweise werden die biologischen Materialien vor der Behandlung mit den organischen Lösungsmitteln in eine lösungsmittelbeständige Umgebung überführt.

In einer weiteren bevorzugten Ausführungsform wird als lösungsmittelbeständige Umgebung ein Gefäß mit einer hydrophoben Oberfläche, insbesondere ein Gefäß mit einer durch Silanisierung hydrophobierten Oberfläche, verwendet.

In einer weiteren Ausführungsform wird ein Gefäß mit einer hydrophoben Oberfläche, insbesondere ein Kunststoffgefäß, vorzugsweise aus Polypropylen, verwendet.

Vorteilhafterweise können im erfindungsgemäßen Verfahren Gefäße mit hydrophoben Oberflächen verwendet. Die hydrophoben Oberflächen lassen sich im Falle von Glasgefäßen durch die Silanisierung der Glasoberfläche oder durch ein Ätzen mit Fluorwasserstoff herstellen. Daneben können auch Kunststoffgefäße, vorzugsweise aus Polypropylen, im erfindungsgemäßen Verfahren verwendet werden. Auch ein Einsatz von Verbundwerkstoffen für die Gefäße, insbesondere kunststoffbeschichtete Gefäße, sind möglich. Bevorzugterweise werden Gefäße verwendet, welche so beschaffen sind, daß sie für spektroskopische Untersuchungen geeignet sind.

In einer weiteren bevorzugten Ausführungsform des Verfahrens werden die Inhaltsstoffe vor der Extraktion in eine lipophile Form überführt und/oder lipophil modifiziert.

Die Erfindung betrifft ferner Verfahren zur Analyse der extrahierten Inhaltsstoffe. Für die Analyse der Inhaltsstoffe kommen sämtliche bekannte oder auch bisher unbekannte Analyse-Techniken in Frage. Eine Trennung der Inhaltsstoffe, beispielsweise durch chromatographische Verfahren, insbesondere mit Hochleistungs-Flüssigkeitschromatographie (high performance liquid chromatography, HPLC), kann sich für die weitere Analyse als förderlich erweisen. Zweckmäßigerweise wird der Überstand analytischen Verfahren zugeführt, welche die Inhaltsstoffe spektrometrisch untersuchen. Als spektrometrische Verfahren können solche in Betracht kommen, welche die Inhaltsstoffe durch eine Wechselwirkung mit elektromagnetischer Strahlung untersuchen, beispielsweise NMR-, IR-, UV-VIS-, Laser-Raman-Spektroskopie. Daneben können sämtliche bekannte massenspektrometische Verfahren zur Anwendung kommen

Eine bevorzugte Ausführungsform der Analyse erfolgt via Spektrophotometer. Besonders bevorzugt ist ein handliches, transportables Gerät, mit welchem man die photometrische Messung schnell und sicher durchführen kann.

Die Erfindung betrifft ferner Analyse-Einheiten enthaltend organischen Lösungsmittel oder Lösungsmittelgemische und deren Verwendung zur direkten Analyse der extrahierten Substanzen.

Besonders bevorzugte Analyse-Einheiten bestehen aus zwei separaten Analyse-Kits, nämlich einem Vorbehandlungs- und und einem Extraktionskit, mit welchen das erfindungsgemässe in zwei Schritten durchgeführt werden kann. So wird das biologische Material in einem Vorbehandlungs-Kit aufgeschlossen und verdünnt. Anschliessend wird eine flüssige Probe aus dem Vorbehandlungs-Kit in einen Extraktions-Kit übergeführt, in welchem dann die Substanz extrahiert, in die organische Phase übergeführt und direkt mit dem Spektrophotometer gemessen wird.

Beide Kits werden dabei beispielsweise durch je ein aus Kunststoff oder Glas gebildetes Gefäss gebildet, welches das vom biologischen Material und der zu analysierenden Substanz abhängige Puffermittel bzw. Lösungsmittel enthält.

Abhängig von den Eigenschaften der Inhaltsstoffe können letztere noch vor der Analyse weiter aufgearbeitet werden, beispielsweise durch Anreicherung und/oder Trennung auf miniaturisierten Kapillaren, die mit Trennmaterialien gefüllt sind.

In einer weiteren bevorzugen Ausführungsform des Analyse-Verfahrens werden die im organischen Lösungsmittel gelösten Inhaltsstoffe direkt angereichert und gleichzeitig getrennt. Die Trennung kann mit aufwendigen chromatographischen Verfahren wie der HPLC oder Gaschromatographie erfolgen oder über reguläre oder miniaturisierte Säulenchromatographie oder Dünnschichtchromatographie. In der bevorzugen Ausführungsform werden die Inhaltsstoffe direkt aus der Extraktionseinheit unter Druck auf einer miniaturisierten Chromatographiesäule angereichert und von störenden Substanzen getrennt. Bei er Anreicherung und Trennung handelt es sich um ein Absorptionsverfahren. Dabei werden die Stoffe durch Van der Waals-Kräfte, Dipol-Dipol-Wechselwirkungen oder Wasserstoffbrücken an der stationären Phase zurückgehalten.

In der bevorzugten Ausführungsform erfolgt Anreicherung und Trennung auf der stationären Phase durch das gleiche Lösungsmittel- oder Lössungsmittelgemisch das zur Extraktion verwendet wurde. Aber auch eine stufenweise Anreicherung und Trennung mit unterschiedlichen Lösungsmitteln oder Lösungsmittelgemischen ist möglich.

In der bevorzugten Ausführungsform befinden sich die zu trennenden und zu untersuchenden lipophilen Komponenten nach dem Extraktionsschritt bereits in der mobilen Phase.

Die Anreicherung und oder Auftrennung erfolgt mittels einer stationären Phase. Als stationäre Phase kommen Kieselgel, Cellulose, Cyklodextrin, Aluminiumoxid, Florisil und andere Substanzen in Frage, die auf Grund ihrer physiko-chemischen Eigenschaften sich für den jeweiligen Inhaltsstoff eignen. Die Auswahl von mobiler und stationärer Phase richtet sich nach dem Trennproblem. Die Materialien können zusätzlich in ihren Oberflächeneigenschaften durch gezielte chemische Modifikationen modifiziert werden. Als Beispiel sei für die Chromatographie von Lipiden die Oberflächenbehandlung von Silikagelen mit Silberionen genannt. Aber auch andere Verfahren können sich eignen.

Neben der Verwendung von einheitlichen stationären Phasen können auch stationäre Phase kombiniert werden. Diese Kombination kann entweder durch Mischung mehrerer verschiedener Phasen erfolgen oder durch einen schichtweisen Aufbau der Säulenfüllung. Durch den schichtweisen Aufbau können verschiedene Trenn- und Anreicherungseffekte erzielt werden.

Der Druckaufbau zum Fluss der mobile Phase kann entweder über die Schwerkraft erzeugt werden oder über andere Verfahren mit denen ein geringer Druck die mobile Phase zum Laufen bringt.

In der bevorzugten Ausführungsform wird der Fluss der mobilen Phase dadurch erzeugt, dass die eine Öffnung einer miniaturisierten mit der stationären Phase gefüllte Säule in die geschlossene Extraktionseinheit verbracht wird. Dieses erfolgt durch Durchstoßen eines Gummiseptums oder ein Septum anderer Art. Ein Abstandshalter an der Säule definiert die Tiefe des Eindringens des Säulchens in das Extraktionsröhrchen. Dadurch kann bei konstantem Probenvolumen und konstantem Lösungsmittelvolumen sichergestellt werden dass sich die Öffnung der Säule im organischen Extraktionsmittel befindet und dass nur eine definierte Menge an Lösungsmittel verwendet wird.

Der Druck für den Fluss des Lösungsmittels wird dadurch aufgebaut, dass mit einer Spritze über eine Nadel neben dem Chromatographieröhrchen eingestochen wird und über die Spritze etwa 10 ml Luft eingepumpt wird. Diese Voilumen ist von der Grösse des Extraktionsröhrchens und dem Volumen des Lösungsmittels abhängig. Durch den geschaffenen Überdruck kommt es zum Fluss der mobilen Phase.

In der bevorzugten Ausführungsform wird auf die gegenüberliegenden Seite ein weiteres leeres Extraktionsröhrchen ausgesetzt, in dem die mobile Phase aufgefangen wird. Dadurch kommt es zu keiner Verschmutzung am Arbeitsplatz. Das ebenfalls vorhandene Gummiseptum schließt sich nach dem Entfernen des Chromatographiesäulchens und beide Einheiten können entsorgt werden ohne dass der Untersucher mit den Chemikalien in Berührung kommt.

Die Identifizierung der Angereicherten und/oder getrennten fettlöslichen Inhaltsstoffe erfolgt entweder direkt mit dem Auge oder mit spektroskopischen Verfahren wenn es sich um Substanzen mit einer charakteristische Eigenfärbung handelt; oder mittels Fluoreszenz wenn die Substanzen über charakteristische Anregungs- und Emissionsspektren verfügen.

Beispiel für fettlösliche Inhaltsstoffe mit charakteristischen Eigenfärbungen finden sich beispielweise in der Gruppe der natürlichen und synthetischen (künstlichen) Farbstoffe. Dazu zählen Carotinoide und Azo-Farbstoffe. Fettlösliche Substanzen mit einer charakteristischen Eigenfluoreszenz sind beispielsweise Vitamin-A-Verbindungen, Vitamin-E-Verbindungen und Mykotoxine.

In einem weitern Schritt können die Substanzen auch modifiziert werden um durch substanzspeifische Reaktionen nachfolgend nachgewiesen werden zu können. Diese Modifikationen können an verschiedenen Stellen des beschrieben Nachweisverfahrens erfolgen. Vor der organischen Extraktion im biologischen Material direkt oder im Material nach oder bei Aufnahme in den Verdünnungspuffer, im organischen Extrakt oder bei oder nach der Auftrennung auf der Chromatographiesäule.

Weitere Einzelheiten und Merkmale der Erfindung ergeben sich aus der nachfolgenden Beschreibung der Darstellung des erfindungsgemäßen Verfahrens und aus bevorzugten Ausführungsformen in Verbindung mit den Unteransprüchen. Hierbei können die jeweiligen Merkmale für sich alleine oder zu mehreren in Kombination miteinander verwirklicht sein.

### Beispiel 1: Bestimmung von Carotenoiden und Vitaminen in Eigelb

Eigelb besteht zu einem grossen Anteil aus Lipiden. Farbgebende Inhaltsstoffe sind die Carotinoide. In Eiern finder man in unterschiedlichen Relationen zu einander die Carotinoide Lutein. Zeaxanthin β-Carotin und Canthaxanthin. Sie gelangen über die Fütterung in das Eigelb. Folgendes Beispiel zeigt den Einfluss der Zusammensetzung der Verdünnungslösung auf die Extrahierbarkeit von Carotinoiden aus Eiern.

Im Rahmen des Vorbehandlungsschrittes werden jeweils 200 mg Eidotter auf das 10-fachen Volumen mit einer Puffer- oder Salzlösung (NaCl-Lösung, Harnstofflösung, Phosphatpufferlösung) bzw destilliertem Wasser gemischt und anschließend in einem einphasigen Lösungsmittelgemisch bestehend aus zwei unterschiedlichen Lösungsmitteln in einem einzigen Schritt isoliert. Die Bestimmung der Carotinoide im organischen Extrakt erfolgte entweder mittels HPLC oder Spektroskopie. Die Bestimmung von Vitamin A und E erfolgte mittels HPLC.
a) Mischung von 200 mg Eigelb mit jeweils 1,8 g Verdünnungslösung entweder destilliertes Wasser oder Puffer- oder Salzlösung (NaCl, PBS oder Harnstoff). Intensive manuelle oder mechanische Mischung (Vorbehandlungsschritt)
b) Aufnahme des Gemisches (in der Regel 400 µl) in einer Spritze und Injektion in eine Spezialküvette über ein Gummiseptum. Spezialküvette enthält ein einphasiges Lösungsmittelgemisch
c) Extraktion der fettlöslichen Komponenten in das Lösungsmittelgemisch durch intensives Schütteln
d) Trennung der Phasen durch Sedimentation für 3 Minuten.
e) Messung des Überstrandes direkt im Spektrophotometer (beispielsweise dem portablen iCheck-Photometer)
Alternativ Abnahme des Lösungsmittelüberstandes und Vermessung der Inhaltsstoffe mittels HPLC oder Spektrophotometrie.

### Beispiel 2: Bestimmung von Carotinoiden (Astaxanthin) in Fischfleisch

Fischmuskel besteht speziesabhängig zu einem großen Anteil aus Fetten unterschiedlicher chemischer Struktur. Farbgebende Inhaltsstoffe im Fischmuskel bei Lachsen und Lachsforellen sind die Carotinoide. Wichtigstes Carotinoid ist das Astaxanthin. Sie gelangen über die Fütterung in das Muskelgewebe. Die Rotfärbung ist ein wesentliches Qualitätsmerkmal für den Verbraucher. Folgendes Beispiel zeigt den Einfluss der Zusammensetzung der Verdünnungslösung (Puffer, Salzlösungen, Harnstofflösung) auf die Extrahierbarkeit von Carotinoiden aus Lachsmuskulatur.

### Schritte

a) Im Rahmen des Vorbehandlungsschrittes werden in einem ersten Schritt zunächst jeweils etwa 3 g Fischmuskelgewebe mittels einer Presse (modifizierte Knoblauchpresse) zerkleinert. Auch andere Möglichkeiten der Zerkleinerung sind denkbar.
b) Anschließend werden jeweils 200 mg zerkleinertes Gewebe in 2 ml destilliertes Wasser oder Pufferlösung bestehnd aus beispielsweise einer NaCl-Lösung, Harnstofflösung oder Phosphatpufferlösung (Verdünnungslösung) aufgenommen und durch intensiver Mixen beispielsweise mit einem modifizierten Milchaufschäumern weiter zerkleinert, aufgeschlossen und für die nachfolgende Extraktion vorbereitet (Vorbehandlungsschritt).
c) Aufnahme des weitgehend homogenen Gemisches (800 µl) in einer Spritze mit 1 ml Volumen und Injektion in eine Spezialküvette über ein Gummiseptum. Spezialküvette enthält ein einphasiges Lösungsmittelgemisch bestehend in der Regel aus zwei verschiedenen organischen Lösungsmitteln.
d) Extraktion der fettlöslichen Komponenten in das Lösungsmittelgemisch durch intensives Schütteln
e) Trennung der Phasen durch Sedimentation für 3 Minuten.
f) Messung des Überstandes direkt im Spektrophotometer (beispielsweise dem portablen iCheck Photometer)
g) Alternativ Abnahme des Lösungsmittelüberstandes und Vermessung der Inhaltsstoffe mittels HPLC oder Spektrophotometrie. Die Bestimmung von Vitamin A und E erfolgte mittels HPLC.

Abbildung 1 zeigt den Einfluss verschiedener Puffer, Lösungsmittel und Harnstoffauf die Extrahierbarkeit von Astaxanthin aus Lachsmuskulatur. Der Vergleich zwischen Abbildung 1 a und b zeigt den Einfluss der Fettkonzentration der Ausgangsprobe. Die Ergebnisse sind als mg/g Fischmuskel dargestellt. In Abhängigkeit von der Fettkonzentration wurden bei vergleichbare Verdünnungslösungen unterschiedliche Ergebnisse erzielt. Am einheitlichsten für beide Matrixes war eine 2 bis 4 M Harnstofflösung.
Verwendete Abkürzungen: Urea = Harnstofflösung; NaCl = Natriumchloridlösung; PBS = Phosphatpuffer in 1-facher bzw 10-facher Konzentrierung)

### Beispiel 3: Stabilisierte Carotinoidpräparate:

Zur Verbesserung der Lager- und Verarbeitungsfähigkeit der empfindlichen Carotinoide werden diese in eine stabilisierende Matrix verpackt.
Folgendes Beispiel zeigt den Einfluß von destiliertem Wasser bzw unterschiedlichen Puffern auf die Extrahierbarkeit der Carotinoide in Abhängigkeit von der Inkubationszeit

Zur Vorbereitung auf de Extraktion wurde ein β-Caroteinpräperat (jeweils 100 mg) mit verschiedenen Puffern, Salz und Harnstofflösungen unterschiedlicer Konzentration und destilliertem Wasser (jeweils 10 ml) über einen Zeitraum von insgesamt 5 Stunden inkubiert (Vorbehandlungsschritt).
a) Inkubation bei Raumtemperatur für 30 min, 1, 2, 3 ,4 und 5 Stunden
b) Zentrifugation der Probe zur Abtrennung der nicht gelösten Komponenten.
c) Abnahme des Überstandes.
d) Verdünnung 1:100 im Ausgangslösungmittel.
f) Aufnahme des Gemisches (400 µl) in einer Spritze (1 ml Volumen) und Injektion in eine Spezialküvette über ein Gummiseptum. Spezialküvette enthält ein einphasiges Lösungsmittelgemisch
g) Extraktion der fettlöslichen Komponenten in das Lösungsmittelgemisch durch intensives Schütteln
h) Trennung der Phasen durch Sedimentation für 3 Minuten.
i) Messung des Überstrandes direkt im Spektrophotometer (beispielsweise in einem portablem Photometer)
j) Alternativ Abnahme des Lösungsmittelüberstandes und Vermessung der Inhaltsstoffe mittels HPLC oder Spektrophotometrie.

Abbildung 2 zeigten den Einfluss unterschiedlicher Verdünnungslösungen bestehend aus Salzen, Puffern oder Harnstoff und Wasser auf die Extraktionseffizienz von Beta-Carotin in stabilisierte Matrix in Abhängigkeit von der Zeit der Inkubation. Die deutliche Effizien von Harnstoff in unterschiedlicher Konzentration (beispielsweise 2 oder 4 M) ist zu sehe. Die Ergebnisse werden als Prozent der maximal erzielten Konzentration und tatsächlichen Konzentration dargestellt.

Verwendete Abkürzungen: Urea = Harnstofflösung; NaCl = Natriumchloridlösung; PBS = Phosphatpuffer in 1-facher bzw 10-facher Konzentrierung)

### Beispiel 4: Extraktion und Bestimmung von Sudan Rot und Carotinoiden aus Eigelb

Zur verbesserung der Gelbfärbung und zur Erhöhung der Farbstabilität werden verschiedenen ntürlicherweise gelb bzw rot farbigen Nahrungsmitteln Farbstoffe zugesetzt. Während die natürlichen oder naturidentischen Carotinoide unbedenklich sind, besteht beim Zusatz von Azoverbindungen ein hohes Maß an gesundheitlichen Risiken. Aus diesem Grunde ist ihr Zusatz verboten und Produkte, die diese künstlichen Farben enthalte, müssen vom Markt genommen werden. Dazu ist ein schneller zuverlässiger und sensitiver Nachweis notwendig.

Ein derartiges Verfahrensbeispile wird nachfolgend an Eigelb beschrieben.

Eigelb besteht zu einem grossen Anteil aus Lipiden. Farbgebende Inhaltsstoffe sind die Carotinoide. In Eiern findet man in unterschiedlichen Relationen zu einander die Carotinoide Lutein. Zeaxanthin β-Carotin und Canthaxanthin. Sie gelangen über die Fütterung in das Eigelb. Zur Intensivierung der Färbung erfolgt die Zufütterung von synthetischen Farbstoffen der Gruppe der Azo-Verbindungen. Ein wichtiger Vertreter ist das Sudan III (rot).

Folgendes Beispiel zeigt die Schritte zur Extraktion und zum Nachweis von Sudan rot aus Eidotter

Jeweils 200 mg Eidotter wurde mit dem 10-fachen Volumen einer Pufferlösung bzw destilliertem Wasser gemischt und anschließend in einem einphasigen Lösungsmittelgemisch in einem einzigen Schritt isoliert. Die Bestimmung der Carotinoide im organischen Extrakt erfolgte entweder mittels HPLC oder Spektroskopie. Die Bestimmung von Vitamin A und E erfolgte mittels HPLC.
a) Mischung von 200 mg Eigelb mit jeweils 1,8 g Verdünner entweder destilliertes Wasser oder Pufferlösung. Intensive manuelle oder mechanische Mischung.
b) Aufnahme des Gemisches (in der Regel 400 µl) in einer Spritze und Injektion in eine spezielle Extraktionseinheit über ein Gummiseptum. Die Extraktionseineheit enthält ein Lösungsmittel oder ein einphasiges Lösungsmittelgemisch
c) Extraktion der fettlöslichen Komponenten in das organische Extraktionsmittel durch intensives Schütteln
d) Trennung der Phasen durch Schwerkraft für 3 Minuten.
e) Optimierung der Phasentrennung durch Zugabe einer hochkonzentrierten Salz-oder Pufferlösung im Volumen von vorzugsweise 500 µl
f) Messung des Überstrandes direkt im Spektrophotometer
g) Alternativ Abnahme des Lösungsmittelüberstandes und Vermessung der Inhaltsstoffe mittels HPLC oder Spektrophotometrie. Die Bestimmung von Vitamin A und E erfolgte mittels HPLC.
h) Zur Anreicherung und Trennung wird an die Extraktion eine säulenchromatographische Trennung angeschlossen. In das Extraktionsröhrchen wird dazu eine mit Silicagel (stationäre Phase) gefüllte miniaturisierte Chromatographiesäule verbracht. Dazu wird das Gummiseptum der Extraktioeinheit durchstossen und das untere eingeführte Ende der Säule so positioniert, dass es sich knapp oberhalb der wässrigen Phase befindet. Auf die Gegenseite des Säulchens wird die Sammeleinheit angebracht.
i) Mittels einer Spritze von 5-10 ml Voliumen wird über eine Nadel Luft ca 10 ml in das Extraktionseinheit über das Septum gepumpt. Es entsthet ein Überdruck, der dazu führt, dass das organische Extraktionsmittel aus der Extraktionseinheit über die Chromatographiesäule (stationäre Phase Silicagel) in die Sammeleinheit fließt. Dabei kommt es zu einer Anreicherung und Trennung der Carotinoide und von Sudanrot.
j) Die semiquantitative Aschätzung der Konzentrationm erfolgt über eine Vergleich der Säule mit der Probe mit Säulen die einen bekannte Konzentration an Sudanrot.
k) Die Detektion erfolgt mit dem Auge oder durch digitale Verstärkung der optischen Signale. Carotiniode und Sudanrot befinden sich in getrennten Banden.

### Beispiel 5: Nachweis von Mykotoxinen

Mykotoxine sind Kontaminanten von Getreide, Getreideprodukten oder beispielsweise Nüssen und Kaffe die von verschieden Pilzen befallen werden. Da sie ein großes gesundheitliches Risiko für Mensch und Tier darstellen
a) Kontaminiertes Getreide (1 g) wurde mit einer 1 ml Pufferlösung (1 Molar Harnstoff) versetzt und für 10 Minuten inkubiert und 5 mal in regelmäßigen Abständen für 10 Sekunden intensiv manuell geschüttelt.
b) Abnahme des wässrigen Überstandes mittels einer Spritze (1 ml) und Injektion von 800 ml in die Extraktionseinheit. Die Extraktionseinheit enthielt ein einphasiges Gemisch aus organischen Lösungsmitteln. Es wurde ein Gemisch von polaren und unpolaren Lösungsmitteln (Ethanol/Isopropanol/Isooctan; 1:4:10) verwendet. Es kommt zu einer sofortigen Phasentrennung. Das Gemisch wird intensiv vorsichtig für 10 Sekunden manuell geschüttelt. Anschließend erfolgt eine Phasentrennung durch Sedimentation für 5 Minuten. Nach 2 bis 3 Minuten wurde erneut geschüttelt und stehengelassen.
k) Messung des Überstrandes direkt im Fluoreszensphotometer
l) Alternativ Abnahme des Lösungsmittelüberstandes und Vermessung der Inhaltsstoffe mittels HPLC oder Fluoreszenzphotometrie.
m) Zur Anreicherung und Trennung wird an die Extraktion eine säulenchromatographische Trennung angeschlossen. In das Extraktionsröhrchen wird dazu eine mit Florisil (stationäre Phase) gefüllte miniaturisierte Chromatographiesäule verbracht. Dazu wird das Gummiseptum der Extraktionseinheit durchstoßen und das untere eingeführte Ende der Säule so positioniert, dass es sich knapp oberhalb der wässrigen Phase befindet. Auf die Gegenseite des Säulchens wird die Sammeleinheit angebracht.
n) Mittels einer Spritze von 5-10 ml Volumen wird über eine Nadel Luft ca. 10 ml in das Extraktionseinheit über das Septum gepumpt. Es entsteht ein Überdruck, der dazu führt, dass das organische Extraktionsmittel aus der Extraktionseinheit über die Chromatographiesäule (stationäre Phase Floresil) in die Sammeleinheit fließt. Dabei kommt es zu einer Anreicherung und Trennung der Mykotoxine.
o) Die semiquantitative Aschätzung der Konzentration erfolgt über eine Vergleich der Säule mit der Probe mit Säulen die einen bekannte Konzentration an Mykotoxinen.
p) Die Detektion der Mykotoxine einer blauen Fluoreszenz erfolgt unter UV-Anregung mit dem Auge oder durch digitale Verstärkung der optischen Signale.

### Beispiel 6 (englisch version): Determination of astaxanthin content in rainbow trout flesh by icheck method in comparison with HPLC.

### Material and methods

The control treatment of the pigmentation trial CP078 in which rainbow trout were fed in triplicate tanks (A1, A2 and A3) a diet supplemented pre-extrusion with 50 ppm astaxanthin as CAROPHYLL Pink 10%-CWS. The final sampling was performed after 12 weeks of experimental feeding. Following the normal procedure for sampling in pigmentation trial, 8 fish were sampled per tank, killed, bled and filleted. The filet was skinned and a 15g flesh sample was taken at the level of the NQC, frozen and given to NRD/CM for HPLC astaxanthin determination according to the standard method. Another smaller flesh sample of ca 5g was taken at the same NQC level and frozen for further icheck determination of astaxanthin. Icheck fish prototypes for astaxanthin determination were provided by BioAnalyt for these tests. The icheck analyses were performed at CRNA according to the protocol established by BioAnalyt which defines a sample size of 0.5g flesh homogenate mixed into 1.5g of buffer in the first phase (Detailed description in Annex 1). The samples were analyzed in duplicates in order to evaluate the reproducibility of the technique.

The technique was then slightly modified in order to increase the sample size and see whether this could improve the accuracy of astaxanthin determination. In this case, the sample size was increased to 0.87g flesh homogenate which were mixed with 2.61 g buffer in order to keep the same dilution rate of the sample and to get to the maximum limit of data input of the spectrophotometer.

### Results

Tables 1 and 2 present the results of measurements of astaxanthin in trout flesh samples of 0.5g by icheck and HPLC, respectively.
Table 3 presents the results of astaxanthin measurements obtained when the size of the sample was increased from 0.5g to 0.87g for samples from fish 6 to 8 in each replicate group.
Table 4 presents the individual and average differences between HPLC and icheck measurements expressed as %, for the two sample sizes. The variation between duplicate measurements is relatively low in most cases.
Results of astaxanthin determination are generally higher by HPLC (Table 2) than by icheck method (Table 1) with samples of 0.5g. Observation was made that in the conditions described in the icheck procedure, the extraction of carotenoids was not complete as some flesh homogenate particles were still pigmented in the icheck vials after three repeated shakings at 5min time intervals. Astaxanthin recovery from icheck extraction procedure was not complete in most cases.
Table 3 shows the results of icheck measurements with flesh samples of 0.87g. This was only done for three fish (fish 6 to 8) per replicate groups. Despite some variations, there is a clear trend for an improved carotenoid recovery with increased sample size. The % differences between icheck and HPLC measurements are in the range of 20 to 30% when the size of the samples was 0.5g (table 4) and they tend to decrease reaching 7% to 18% as average between replicate groups with samples of 0.87g. The variations are bigger with the flesh samples of 0.87g due to the lower number of samples analyzed.

Although the method developed for quick analysis of astaxanthin works well, improvement in terms of recovery of carotenoid through icheck procedure are required in order to lower the % difference with H PLC to below 10% on a constant basis.

**Table 1: Astaxanthin contents in mg/kg of trout flesh measured with icheck using 500 mg sample. Values represent individual measurements of 8 fish per replicate tanks in duplicates.**

| | | **A1** | | **A2** | | **A3** | |
|---|---|---|---|---|---|---|---|
| **Fish** | **Rep.** | **Asta mg/kg** | **Means ± SD** | **Asta mg/kg** | **Means ± SD** | **Asta mg/kg** | **Means ± SD** |
| 1 | 1a | 6,40 | 7.65 ± 1.76 | 6,35 | 7.59 ± 1.75 | 7,70 | 8.18 ± 0.67 |
| | 1b | 8,89 | | 8,83 | | 8,65 | |
| 2 | 2a | 4,66 | 5.99 ± 1.87 | 7,15 | 7.32 ± 0.23 | 6,61 | 7.07 ± 0.65 |
| | 2b | 7,31 | | 7,48 | | 7,53 | |
| 3 | 3a | 7,37 | 7,77 ± 0.57 | 9,34 | 9.34 ± 0.00 | 8,05 | 8.32 ± 0.38 |
| | 3b | 8,17 | | 9,34 | | 8,59 | |
| 4 | 4a | 7,09 | 7,57 ± 0.68 | 7,65 | 8.56 ± 1.29 | 7,59 9,02 | 8.31 ± 1.01 |
| | 4b | 8,05 | | 9,47 | | | |
| 5 | 5a | 9,93 | 9,70 ± 0.33 | 7,99 | 8.67 ± 0.95 | 8,83 9,21 | 9.02 ± 0.27 |
| | 5b | 9,47 | | 9,34 | | | |
| 6 | 6a | 8,23 | 8,32 ± 0.13 | 6,82 | 7.53 ± 1.00 | 8,47 | 9.44 ± 1.37 |
| | 6b | 8,41 | | 8,23 | | 10,41 | |
| 7 | 7a | 5,80 | 6,42 ± 0.88 | 8,29 | 9.08 ± 1.11 | 9,60 | 10.01 ± 0.57 |
| | 7b | 7,04 | | 9,86 | | 10,41 | |
| 8 | 8a | 8,89 | 9,41 ± 0.74 | 10,20 | 10.41 ± 0.30 | 7,26 6,98 | 7.12 ± 0.20 |
| | 8b | 9,93 | | 10,62 | | | |

**Table 2. Astaxanthin content in mg/kg of trout flesh measured with HPLC. Values represent individual measurements of 8 fish per replicate tanks.**

| **HPLC (Asta mg/kg)** | | | |
|---|---|---|---|
| **Fish** | **A1** | **A2** | **A3** |
| 1 | 10,69 | 10,12 | 11,21 |
| 2 | 10,44 | 9,92 | 9,08 |
| 3 | 10,78 | 12,10 | 11,00 |
| 4 | 13,38 | 10,85 | 10,10 |
| 5 | 12,18 | 12,09 | 9,65 |
| 6 | 11,60 | 9,08 | 11,20 |
| 7 | 8,60 | 11,57 | 12,46 |
| 8 | 11,45 | 11,74 | 9,27 |
| Mean | **11,14** | **10,93** | **10,50** |
| SD | **1,40** | **1,13** | **1,16** |

**Table 3: Astaxanthin contents of trout flesh measured with icheck with flesh samples of 0.87g**

| | | **A1** | | **A2** | | **A3** | |
|---|---|---|---|---|---|---|---|
| **Fish** | **Reps** | **Asta mg/kg** | **Means ± SD** | **Asta mg/kg** | **Means ± SD** | **Asta mg/kg** | **Means ± SD** |
| 6 | 6a | 11,29 | | 8,42 | | 10,15 | |
| | 6b | 9,94 | 10.62 ± 0.95 | 8,00 | 8.21 ± 0.30 | 9,48 | 9.82 ± 0.47 |
| 7 | 7a | 8,66 | | 6,89 | | 8,24 | |
| | 7b | 7,55 | 8.11 ± 0.78 | 10,92 | 8.91 ± 2.85 | 8,12 | 8.18 ± 0.08 |
| 8 | 8a | 11,82 | | 10,49 | | 8,30 | |
| | 8b | 9,61 | 10.72 ± 1.56 | 10,56 | 10.53 ± 0.05 | 11,51 | 9.91 ± 2.27 |

**Table 4: Percentages of differences between the results of HPLC and icheck methods for the two flesh sample sizes (HPLC = 100%)**

| | **A1** | | **A2** | | **A3** | |
|---|---|---|---|---|---|---|
| **Fish** | **0.5g (%)** | **0.87g (%)** | **0.5g (%)** | **0.87g (%)** | **0.5g (%)** | **0.87g (%)** |
| 1 | 28,44 | - | 25,00 | - | 27,03 | - |
| 2 | 42,62 | - | 26,21 | - | 22,14 | - |
| 3 | 27,92 | - | 22,81 | - | 24,36 | - |
| 4 | 43,42 | - | 21,11 | - | 17,72 | - |
| 5 | 20,36 | - | 28,29 | - | 6,53 | - |
| 6 | 28,28 | 8,45 | 17,07 | 9,58 | 15,71 | 12,32 |
| 7 | 25,35 | 5,70 | 21,52 | 22,99 | 19,66 | 34,35 |
| 8 | 17,82 | 6,38 | 11,33 | 10,31 | 23,19 | -6,90 |
| **Mean** | **29.28** | **6.84** | **21.67** | **14.29** | **19.54** | **17.86** |
| **SD** | **9.32** | **1.43** | **5.41** | **7.54** | **6.40** | **14.54** |

### Weitere Figuren Beschreibungen

Figur 3 zeigt den Einfluss unterschiedlicher Vorbereitungspuffer auf die Extraktion von Sudan Rot aus Eigelb in einen organischen Überstand aus n-Hexan. Es gilt:
1 = Wasser (Verdünnungslösung); n-Hexan (Extraktionslösungsmittel)
2 = 10% DMSO + 1 M Harnstoff (Verdünnungslösung); n-Hexan (Extraktionslösungsmittel)
3 = 25 % DMSO + 1 M Harnstoff (Verdünnungslösung); n-Hexan (Extraktionslösungsmittel)
4 = 50% DMSO + 1 M Harnstoff (Verdünnungslösung); n-Hexan (Extraktionslösungsmittel)
5 = 75% DMSO + 1 M Harnstoff (Verdünnungslösung); n-Hexan (Extraktionslösungsmittel)
6 = 1 M Harnstoff (Verdünnungslösung); Ethanol:iso-Propanol:n-Hexan (volumenanteile 1:2:6) (Extraktionslösungsmittel)

Figur 4 zeigt den Aufbau des Einweg-Analyse- Kits zur Anreicherung durch Extraktion und nachfolgenden Anreicherung und Trennung von fettlöslichjen Inhaltsstoffen bestehend aus der Extraktionseinheit, der Sammeleinheit und der miniaturisierten Chromatographiesäule.

Figur 5 zeigt die mit dem Auge erkennbare Färbung der stationären Phase durch Carotinoide (Pfeil A) und Sudanrot (Pfeil B) nach der direkten Elution mit der organischen Extraktionsphase aus Abbildung 3 (n-Hexan) als mobilen Phase Der Vergleich von Figure 4a und 4b zeigen die Möglichkeit der Signalverstärkung mittels Digitalisierung. In Fugure 4b wurde eine digitale Verstärkung der optischen Signale durchgeführt.

## Patentansprüche

1. Verfahren zur Extraktion von Inhaltsstoffen, insbesondere von Lipiden und lipoiden Substanzen aus biologischem Material, **gekennzeichnet durch** einen Vorbehandlungsschritt, der dazu dient den Inhaltsstoff für die Extraktion leichter verfügbar zu machen und einer nachfolgenden Extraktion in ein organisches Lösungsmittel bestehend aus einem einphasigen Lösungsmittelgemisch, das sich **durch** die Zugabe der wässrigen Probe in zwei Phasen teilt und in der organischen Lösungsmittelphase die Inhaltsstoffe nachgewiesen werden können.

2. Verfahren nach Anspruch 1, **dadurch gekennzeichnet dass** es sich bei den I ipophilen Stoffen um natürliche oder synthetische (künstliche) fettlösliche Farbstoffe handelt,

3. Verfahren nach Anspruch 2, **dadurch gekennzeichnet, dass** es sich bei den Farbstoffen um Carotenoide wie etwa Astaxanthin, Canthaxanthin, Beta-Carotine oder Apo-Ester handelt.

4. Verfahren nach Anspruch 2, **dadurch gekennzeichnet, dass** es sich bei den synthetischen Farbstoffen um Azoverbindungen handelt.

5. Verfahren nach einem der vorhergehenden Ansprüche, **dadurch gekennzeichnet, daß** als biologische Materialien Lebensmittel oder Futtermittel tierischen und/oder pflanzlichen Ursprungs, insbesondere Homogenisate von Lebensmitteln oder Futtermitteln, eingesetzt werden.

6. Verfahren nach einem der vorhergehenden Ansprüche, **dadurch gekennzeichnet, dass** als Vorbehandlung ein spezifischer Verdünnungsschritt dient, der gegebenenfalls, d.h. vor allem bei festen biologischen Materialien, noch zusätzlich mit einem Aufschluss-Schritt kombiniert ist.

7. Verfahren nach einem der vorhergehenden Ansprüche, **gekennzeichnet durch** eine Vorbehandlung mit bestimmten Salz- oder Pufferlösungen insbesondere Harnstofflösungen unterschiedlicher Konzentration.

8. Verfahren nach einem der vorhergehenden Ansprüche, **dadurch gekennzeichnet, daß** für die Extraktion als organisches Lösungsmittel polar protische Lösungsmittel verwendet werden und dass die polar protischen Lösungsmittel ausgewählt sind aus der Gruppe, umfassend Methanol, Ethanol, 1-Propanol, 2-Propanol (Isopropanol), Butanol, Pentanol, Hexanol sowie Gemische davon.

9. Verwendung des Verfahrens nach einem der vorhergehenden Ansprüchen zur Analyse von Inhaltsstoffen biologischer Materialien, wobei die Analyse zum Nachweis von Farbstoffen in Nahrungs- und Futtermitteln eingesetzt wird.

10. Verwendung nach Anspruch 9, **dadurch gekennzeichnet, daß** die Analyse zur Verfolgung einer Farbverfälschungen in Nahrungs- und Futtermitteln eingesetzt wird.

11. Verwendung nach Anspruch 9 oder 10 zur Analyse von Carotiniden in Fisch.

12. Verwendng nach Anspruch 9 oder 10 zur Analyse von Farbstoffen in Eier oder Eihaltigen Produkten.

13. Analyse-Einheit zur Durchführung des Verfahrens nach einem der vorhergehenden Ansprüchen, bestehend aus einem Vorbehandlungs-Kit und einem Extraktions-Kit, wobei beide Kits je ein vom biologischen Material und der zu analysierenden Substanz abhängiges Lösungsmittel bzw. Lösungsmittelgemisch der vorstehend definierten Art enthalten.

14. Analyse-Einheit nach Anspruch 13 zum Nachweis von Farbstoffen in Nahrungs- und Futtermitteln.

15. Spektrophotometer, insbesondere Handphotometer, zur Messung von Inhaltsstoffen von biologischen Materialien in einem den Extraktions-Kit gemäss Anspruch 13 bildenden durchsichtigen Gefäss, **dadurch gekennzeichnet, dass** der Strahlengang des Photometers so eingestellt ist, dass die Messung die obere Hälfte des Gefässes erfasst.
